# EUROPEAN PATENT SPECIFICATION

(11) **EP 3 081 217 B1**
(45) Date of publication and mention of the grant of the patent: **28.10.2020**
(21) Application number: 16165097.3
(22) Date of filing: 13.04.2016
(51) Int. Cl.: A61K 31/55, A61K 47/34, A61K 9/00, A61B 17/12, A61P 9/14

(54) **INJECTABLE ALBUMIN BASED VASCULAR OCCLUDER**
INJIZIERBARE ALBUMINBASIERENDE VASKULÄRE OKKLUSIONSVORRICHTUNG
DISPOSITIF D'OCCLUSION VASCULAIRE À BASE D'ALBUMINE INJECTABLE

(30) Priority: 13.04.2015 US 201562146611 P
(43) Date of publication of application: 19.10.2016
(73) Proprietor: Cook Medical Technologies LLC, Bloomington, IN 47404 (US)
(72) Inventor: TANNER, Shaun Anthony, West Lafayette, Indiana 47906 (US)
(74) Representative: Mathys & Squire

(56) References cited:
- WO-A1-00/12018
- US-A1- 2001 051 813
- US-A1- 2008 050 436
- US-A1- 2009 202 467
- MARK PREUL: "Recombinant Human Albumin PEG Hydrogel with Dual Functions: Sealing CSF Leak and Mitigating Peridural Fibrosis in a Large Animal Durotomy", THE SPINE JOURNAL, vol. 12, no. 9, 1 September 2012 (2012-09-01), pages S160-S161, XP055282505, AMSTERDAM, NL ISSN: 1529-9430, DOI: 10.1016/j.spinee.2012.08.411
- D'URSO E M ET AL: "NEW BIOARTIFICIAL POLYMERIC MATERIAL: POLY(ETHYLENE GLYCOL) CROSS-LINKED WITH ALBUMIN. I. SYNTHESIS AND SWELLING PROPERTIES", JOURNAL OF BIOACTIVE AND COMPATIBLE POLYMERS, LANCASTER, PA, US, vol. 9, no. 4, 1 October 1994 (1994-10-01) , pages 367-387, XP000606152,

## Description

### BACKGROUND

The present disclosure relates to medical devices and compositions of matter for treatment. More particularly, the disclosure relates to a device for occlusion and treatment of a body vessel. In one embodiment the present disclosure relates to the reversible occlusion and treatment of varicose and spider veins. In another embodiment, the present disclosure relates to treatment of arteriovenous malformations.

Varicose veins and spider veins are venous blood vessels that have become enlarged and twisted due to weakened vessel walls. Veins have leaflet valves to prevent blood from flowing backwards (that is, retrograde). When veins become varicose, the leaflets of the valves no longer meet properly, and the valves do not work, a condition which is known as valvular incompetence. Blood then flows backwards and the veins continue to enlarge.

Varicose veins are most common in the superficial veins of the legs, which are subject to high pressure when standing. Besides being a cosmetic problem, varicose veins can be painful, particularly when standing. Severe long-standing varicose veins can lead to leg swelling, venous eczema, skin thickening (lipodermatosclerosis) and ulceration.

Typically, varicose and spider veins are treated by sclerotherapy. In a sclerotherapy procedure, a sclerosing agent is delivered to the site. The sclerosing agent causes the vessel wall to undergo fibrosis and the vessel eventually shrinks. In one sclerotherapy protocol, a solution of sodium tetradecyl sulfate (STS) dissolved in water to form a liquid treatment solution. In other applications, such a solution is mixed with air or with a physiological gas such as carbon dioxide. The mixing action generates a foam. The foam can be loaded into a syringe or, alternatively, formed therein. The foam is then injected into the varicose vein.

However, such a treatment is not without its drawbacks. Because blood is still flowing through the vein at the site of injection, a large amount of the sclerosant foam can be carried away from the site to be treated. As such, a larger amount of foam and therefore active ingredient, such as STS, must be used to treat the patient.

There is a need for an improved treatment for varicose and spider veins which will increase the efficiency of the treatment, shrink problematic vessels, and use less sclerosant.

US 2001/0051813 discloses compositions for closing vascular puncture sites.

US 2008/0050436 discloses a method for obliterating affected vessels.

US 2009/0202467 discloses compositions for treating varicose veins.

WO 00/12018 discloses a biodegradable material for application to tissue.

Preul (Spine Journal, 2012, 12, 9) discloses hydrogels for sealing CSF leaks and mitigating peridural fibrosis.

D'Urso et al. (J. Bioactive and Compatible Polymers, 1994, 9, 4) disclose hydrogels obtained by cross-linking poly(ethylene glycol) with albumin.

### SUMMARY

The subject matter of the present invention is defined by the appended claims.

More generally, the present disclosure provides, in a first aspect, a composition of matter for injection into and occlusion of a body vessel is provided comprising a polymer compound, a protein comprising a sulfur-bearing amino acid, and a sclerosant, wherein the composition of matter is substantially fluid at a first temperature and substantially non-fluid at a second temperature, the first temperature being lower than the second temperature.

In another aspect of the present disclosure, a composition of matter for injection into and occlusion of a body vessel is provided, the composition comprising: a nonionic copolymer comprising a first chain having a first end and a second end, a second chain having a first end and a second end, and a third chain having a first end and a second end, the second end of the first chain being bound to the first end of the second chain, the second end of the second chain being bound to the first end of the third chain, the first chain and the third chain comprising poly(ethylene oxide) and together comprising about seventy percent of a molecular mass of the polymer, the second chain comprising poly(propylene oxide) and having a molecular mass of about 4000, a quantity of an albumin, and a sclerosant wherein the quantity of albumin and the nonionic copolymer are crosslinked to effect occlusion of the vessel.

According to another aspect of the present disclosure, there is provided a method of making a composition for occlusion of a body vessel comprising the steps of: treating a quantity of albumin with a reducing agent to form a solution of albumin-SH; treating a quantity of a nonionic polymer with dichloromethane, sodium hydride, and divinyl sulfone to form a solution of polymer-DVS; combining the albumin-SH with the polymer-DVS and a quantity of sclerosant to form a hydrogel.

According to a further aspect of the present disclosure, there is provided a method of treating varicose or spider veins with an injectable, thermoreversible occluder, wherein the occluder is a hydrogel comprising a sclerosant, a sulfur-bearing protein, and a polymer, the occluder being a liquid at a first lower temperature and a hydrogel at body temperature.

According to a further aspect of the present disclosure, there is provided a composition of the present disclosure for use in therapy and, in particular, for use in a method of treating varicose veins or spider veins in a patient. In an embodiment, the composition is delivered to a patient requiring treatment of varicose veins. In an embodiment, the composition is removed from the patient after treatment of varicose veins has concluded.

Further aspects, features, and advantages of the disclosure will become apparent from consideration of the following description, especially when viewed in conjunction with the drawings and the appended claims.

### BRIEF DESCRIPTION OF THE DRAWINGS

FIG. 1 is an illustration of the structure of a single type of polymer which can be used in accordance with one embodiment of the present disclosure;
FIG. 2 is a scheme illustrating a specific procedure for synthesizing a particular occlusive composition in accordance with the principles of the present disclosure;
FIGs. 3A and B are graphical representations of rheological data illustrating properties of the occlusive composition generated by a reaction scheme according to the principles of the present disclosure; and
FIGs. 4A, 4B, and 4C are photographs of the vascular anatomy of a pig (4A) untreated, (4B) one minute after treatment with an occlusive composition of the present disclosure, and (4C) five minutes after treatment with an occlusive composition of the present disclosure.

### DETAILED DESCRIPTION

The term "substantially" used herein with reference to a quantity includes variations in the recited quantity that are equivalent to the quantity recited, such as an amount that is equivalent to the quantity recited for an intended purpose or function. The term "substantially fluid" encompasses a material which is completely or mostly in a fluid state, but some portions of the material may have partially or completely solidified. The term "substantially nonfluid" encompasses a material which is completely or mostly in a nonfluid (that is, solid or semisolid) but may have some fluid characteristic. A gel is a nonfluid which is expanded by a fluid. In a hydrogel, said fluid comprises water.

The term "about" when used in the context of a numerical value or range set forth means a variation of ±25%, or less, of the numerical value. For example, a value differing by ±25%, ±20%, ±15%,±14%,±10%, or ±5%, among others, would satisfy the definition of "about." When a temperature is recited, "about" may also refer to a temperature variation of ±10 degrees Celsius, or ±5 degrees Celsius, or ±2 degrees Celsius, or ±1 degree Celsius, or ±0.5 degree Celsius.

As used in this disclosure, the term "polymer" means a natural or synthetic molecule or macromolecule which comprises repeated subunits.

As used in this disclosure, the term "copolymer" means a natural or synthetic polymer which is made up of one or more types of monomeric subunits repeated in any way.

As used in this disclosure, a "co-block polymer" or "block copolymer" refers to a natural or synthetic copolymer in which a chain having a first end and a second end and comprising a first monomer is attached to a chain having its own first end and extending to a second end and comprising a second monomer. The chains are then joined end-to-end; for instance, the second end of the first chain may be bound to the first end of the second chain, or some other arrangement. Prefixes may denote the number of types of monomers or may denote the number of chains. For instance, a triblock copolymer may comprise chains of three different monomeric subunits, or may comprise a first chain of monomer A, a chain of different monomer B attached at its first end to the first chain of monomer A, and then another (second) chain of monomer A attached at its first end to the second end of the chain of monomer B to form an A-B-A chain structure.

As used in this disclosure, the term "sulfur-bearing amino acid" means any amino acid which contains at least one atom of sulfur, including but not limited to cysteine and methionine.

As used in this disclosure, the term "sclerosant" means any substance, generally comprising an irritant, injected into the lumen of a vein in order to affect the destruction of the target vessel. The term "sclerosant" is interchangeable with the term "sclerosing agent."

As used herein, the term "occlusion" may refer to complete occlusion, or any degree of partial occlusion.

As used in this disclosure, the term "thermoreversible" means "changeable or modifiable by heating." Thermoreversible substances have one property at a first temperature and a different property at a second, different temperature.

Presently used methods of treating cosmetically-undesirable and painful varicose and spider veins are in need of advancement. In one prior art example, the sclerosing agent sodium tetradecyl sulfate (STS) is dissolved in water and mixed with air to create a foam. This foam is delivered via syringe to the target vessels. However, blood continues to flow through the veins during treatment, and the foam including the active sclerosant is motile; that is, it is not retained at the site to be treated. In turn, more sclerosant than the active dose, sometimes much more, must be used in order to treat problem veins.

Described herein is a therapeutic composition of matter which serves a dual role: briefly, a thermoreversible composition which is capable of solidifying at body temperature to form a hydrogel, including but not limited to an exemplary composition whose synthetic scheme follows, functions both as an occluder to disrupt blood flow and minimize migration of the agent, and to retain sclerosant in a matrix and gradually release said sclerosant from the matrix at the site to which it was delivered. Further aspects of an exemplary composition described herein include thermoreversible properties, wherein at a first cooler temperature such as about 32 degrees Celsius the composition is substantially liquid and at a higher temperature, such as human body temperature or about 37 degrees Celsius, the composition is substantially a solidified, non-fluid hydrogel. Finally, the exemplary composition is non-toxic and can be retained within the vasculature of a patient for a relatively great length of time.

In one aspect, such an occluder can be a tripartite composition: the first part can be a polymer, the second part can be a protein or polypeptide, and the third part can be a molecule to be delivered, such as at least one sclerosant, or at least one other water-soluble substance.

In one embodiment, the polymer is a copolymer having more than one type of monomeric subunit. In a further embodiment, the copolymer can be a block copolymer, and the polymer may comprise or consist of a non-ionic copolymer which may have hydrophilic portions, hydrophobic portions, or both.

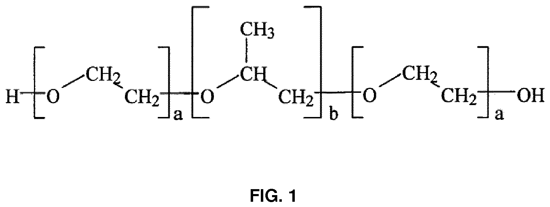

In one example, the polymer may be a poloxamer. In one embodiment, the poloxamer may be a triblock copolymer comprising a central hydrophobic block of polypropylene glycol (also known as polypropylene oxide) flanked by two hydrophilic blocks of polyethylene glycol (PEG) on both sides of the hydrophobic block. In one example, a poloxamer known as Poloxamer 407 (P407) may be used. Such a polymer has two PEG blocks of about 101 repeated units flanking about 56 units of propylene glycol. Examples of this compound include BASF PLURONIC F127 and Croda SYNPERONIC PE/F 127. A structure of P407 is illustrated in FIG. 1 wherein the number of monomers chained together to form a PEG is represented by a quantity a and the number of monomers chained together form a chain of propylene glycol is represented by a quantity b.

Poloxamer 407 is a nonionic triblock copolymer comprising a first block, a second block, and a third block, each block comprising a polymer chain having a first end and a second end, wherein the second end of the first block is bound to the first end of the second block, the second end of the second block is bound to the first end of the third block, and the first end of the first block and the second end of the third block are free ends. The second block comprises a chain of poly(propylene oxide). In this embodiment the chain of poly(propylene oxide) has a molecular mass of about 4000. The first block and the third block each comprise a chain of poly(ethylene oxide), the first block and the third block together comprising about seventy percent of the weight of the polymer, or a combined molecular mass of about 9333.

Other poloxamers, having varying ratios of propylene glycol to PEG, may also be used. It is envisioned that many other types of natural and synthetic polymers can be utilized in the synthesis of a hydrogel capable of performing an occlusive and therapeutic function, including but not limited to compositions comprising a polyethylene, a polypropylene, a polystyrene, a polytetrafluoroethylene, a polyvinylchloride, a polychlorotrifluoroethylene, and many other types of natural and synthetic polymers. The identity and concentration of polymers may change the properties of the resulting hydrogel. As mentioned, the occlusive composition in accordance with the principles of this disclosure includes both a polymeric and a polypeptide or protein component. Increasing the relative amount of a certain polymer relative to the amount of polypeptide or protein used may increase the transition temperature of the composition, whereas increasing the relative amount of a polymer having a different monomeric composition may instead decrease the transition temperature. Other factors which may be changed when various combinations of polymers and proteins or polypeptides are mixed may yield hydrogels with lesser resistance to blood flow, or incomplete solid-to-liquid transitions at body temperature, or both.

Some poloxamers, such as poloxamer 407, belong to a class of molecules known as reverse thermosensitive polymers. Such molecules have a property wherein they behave oppositely of expectations; instead of becoming more fluid at higher temperatures and more solid at lower temperatures, these polymers become more solid at higher temperatures and more fluid at lower temperatures.

The final concentration (weight-to-volume) of polymer can vary based on the identity of the polymer used. For instance, a very large polymer with a great number of active groups may only be present at about 5% weight-to-volume whereas a small polymer with a single active group may make up 95% of the composition of matter. In one embodiment, the polymer makes up about 5% to about 95% of the composition of matter. In another embodiment, the polymer comprises about 7.5% to about 85% of the composition. In another embodiment, the polymer comprises about 10% to about 70% of the composition. In another embodiment, the polymer comprises about 10% to about 50% of the composition. In another embodiment, the polymer comprises about 10% to about 30% of the composition of matter. In another embodiment, the polymer comprises about 10% to about 20% of the composition of matter. In another embodiment the polymer comprises about 12.5% to about 15% of the composition of matter.

A second component of an occlusive composition in accordance with the principles of this disclosure can be a polypeptide or a protein. In one embodiment, a polypeptide or a protein which contains at least one sulfur-bearing amino acid may be employed. In another embodiment, a polypeptide or protein lacking a sulfur-bearing amino acid may be employed, wherein said polypeptide or protein has been engineered to generate a site containing a sulfur, particularly a free thiol.

Sulfur-containing amino acids include but are not limited to methionine, cysteine, homocysteine, and taurine. A polypeptide or protein incorporating a selenium-bearing amino acid, such as for example selenocysteine, may also be employed. In particular, proteins with free cysteine are useful in practicing the disclosure described herein.

Amino acids containing free sulfur (or selenium), in particular cysteine, are capable of forming disulfide (or diselenide) bonds in their oxidized form. Methionine is less useful in this regard but methionine can be modified to eliminate the terminal alkyl group of the side chain and thereby free a sulfur for creation of a thiol site.

Disulfide bonds are covalent bonds which, in some cases, impart tertiary or quaternary structure to a polypeptide, protein, or protein complex. However, in reduced form, these bonds break and the constituent amino acid side chains instead yield free thiol groups, which are in turn able to form other covalent bonds. Thus, the sulfur of a side chain of an amino acid can be used as a reactive group for creating bonds to other groups.

In one embodiment, a family of proteins that may be employed as some or all of the protein constituent of an occlusive composition of matter is the albumin family. For the purpose of brevity, "albumin" as used herein can refer to any protein which is considered an albumin alone, or a combination of any number of albumin family proteins, including but not limited to serum albumins.

Albumin is a water-soluble protein which has an overall globular structure. Albumin in the form of serum albumin is the most abundant protein found in mammalian blood serum and is generally not glycosylated. One particularly well-studied albumin is bovine serum albumin (BSA), which is easily obtained due to the large quantity of this protein found in bovine blood, a waste product of the cattle industry. The mature BSA protein contains 583 amino acids, 35 of which are cysteine residues which naturally form 17 disulfide bonds among themselves. Albumins are known to contain a proportionately high number of cysteine residues per molecule, which increases their suitability for use in the present disclosure. Although a polymer, such as a poloxamer, may on its own be capable of forming a thermoreversible hydrogel, crosslinking of a protein such as albumin to a polymer such as a poloxamer, and in particular poloxamer 407, improves the resistance of the resultant hydrogel against pulsatile blood flow and enhances the ability of the hydrogel to adhere to the wall of the vessel into which it is injected.

The amount of polypeptide or protein present (weight to volume) in the composition of matter can vary based on the molecular weight of the protein, the number and availability of cysteine side chains, and other factors. In one embodiment, the protein or polypeptide can comprise about 0.5% to about 70% of the composition of matter, weight-to-volume. In another embodiment, the protein can comprise about 1% to about 50% of the composition of matter, weight-to-volume. In another embodiment, the protein comprises about 2% to about 40% of the composition of matter, weight to volume. In another embodiment, the protein comprises about 3% to about 30% of the composition of matter, weight-to-volume. In another embodiment, the protein comprises about 4% to about 20% of the composition of matter, weight-to-volume. In another embodiment, the protein comprises about 5% to about 10% of the composition of matter, weight-to-volume.

The third component of the tripartite composition of the present disclosure is a sclerosant, the active agent for sclerosis and treatment of varicose and spider veins. Sclerosants which can be used in accordance with an embodiment of the present disclosure include, but are not limited to, STS, 7-ethyl-2-methyl-4-undecanyl sulfate sodium salt, and polidocanol.

A general scheme for synthesizing an occlusive composition according to the principles of the present disclosure is briefly described. In a first step 10, a protein is treated with a reducing agent to generate a reduced protein having free thiol groups. In a second step 20, a polymer is activated and functionalized, in some cases comprising a vinyl sulfone. A third step 30 comprises combining the reduced protein, the activated polymer, and a sclerosant in any order to form the occlusive compound. For instance, in third step 30, the three components may be stored separately until just prior to treatment, or the reduced protein can be mixed with the sclerosant and stored, or the activated polymer may be combined with the sclerosant and stored, or the activated polymer and the reduced protein can be mixed with the sclerosant and stored, or all three components may be mixed together and stored until needed for treatment of a patient.

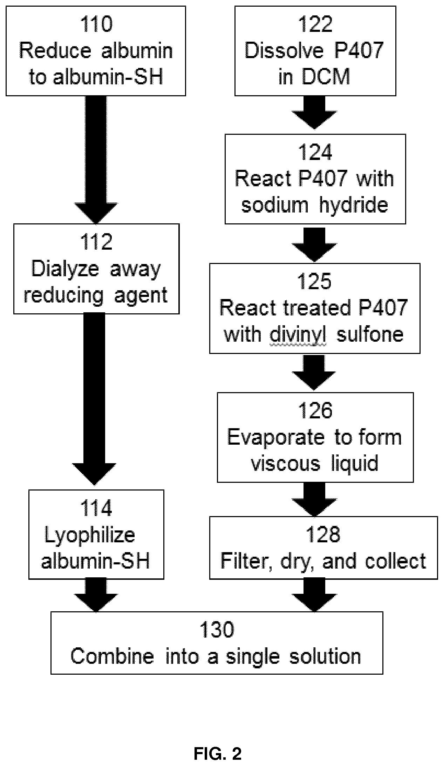

FIG. 2 illustrates the steps of synthesis of an exemplary occlusive and therapeutic composition of matter according to the principles of the present disclosure. This example is merely illustrative and is not intended to be limiting. The exemplary occlusive composition has thermoreversible properties, including a transition temperature at about 32 degrees Celsius, a substantially liquid state at temperatures cooler than the transition temperature, and a substantially solid state at temperatures higher than the transition temperature, including at about human body temperature (about 37 degrees Celsius).

In a first step 110, albumin is reduced to albumin-SH. An albumin such as a serum albumin, in one embodiment BSA, is dissolved in deionized water to a final concentration of about 6.25% (weight/volume). The pH of the resulting solution is adjusted to about 8 with a solution of sodium hydroxide (in this case, 1 molar (M).) A reducing agent, in one embodiment DL-dithiothreitol (*threo*-1,4-Dimercapto-2,3-butanediol, or DTT), is added to a final concentration of 10% (weight/weight) to the albumin and the solution is stirred under a nitrogen atmosphere for about 3 hours, though longer and shorter stirring times may also be acceptable. In this step, the disulfide bonds in the albumin are converted to free thiol (-SH) groups. Other reducing agents may be employed to convert the disulfide bonds to free thiols, including but not limited to beta-mercaptoethanol, Tris (2-carboxyethyl) phosphine (particularly in the form of Tris (2-carboxyethyl) phosphine hydrochloride), and dithiobutylamine (DTBA).

The resultant albumin is referred to as albumin-SH. To remove excess reducing agent, in optional step 112 the albumin-SH can be optionally dialyzed against deionized water in a membrane having about a two kilodalton molecular weight cutoff for about 48 hours. The dialysis regimen can be adjusted to longer times, and may also be effective in removing excess reducing agent in under about 48 hours, particularly if the deionized water used outside of the membrane is changed frequently. In optional step 114, the albumin-SH can then be lyophilized, collected, and stored at about 4 degrees Celsius.

A second part in the preparatory scheme of the exemplary embodiment includes adding a functional group to at least one of the polymers to be employed. In this exemplary embodiment, the polymer is poloxamer 407 (P407) but many other polymers can be used. The reactive group generated in this scheme is a divinyl sulfone group. In a step 122, about 10 grams (about 0.8 millimole (mmol)) of P407 is dissolved by stirring in about 30 milliliters (ml) of dichloromethane (DCM) at room temperature under a dry nitrogen atmosphere. About 0.19 grams of sodium hydride (about 8 mmol, or about a 5X molar excess) is then dissolved in about 15 ml DCM. In a step 124, the P407 and sodium hydride solutions are combined and reacted for about an hour under a dry nitrogen atmosphere. In a step 125, the P407-sodium hydride combination is then added dropwise to a mixture of about 9.4 grams of divinyl sulfone (about 80 mmol or about 50X molar excess) and about 20 ml DCM, stirring in the dark under dry nitrogen for about 72 hours, though greater and lesser times may be sufficient for the reaction to go to completion. Volatiles are then removed, and the volume of the solution is decreased, in a rotary evaporator to produce a viscous liquid in a step 126. The resultant viscous liquid can then be combined in a step 127 (not shown) with a volume of ice-cold (about 0 degrees Celsius, plus or minus several degrees) diethyl ether. In a step 128, the mixture is filtered and the solid product is collected and dried under reduced pressure. This solid is Poloxamer 407-divinyl sulfone (P407-DVS) and is then stored at about 4 degrees Celsius.

Different reactive groups can be achieved in other ways. For example, hydroxyl groups can serve as starting points for vinyl sulfones. Acryl groups, rather than thiols, of a protein or polypeptide may also be exploited. However, using another method might affect thermoreversibility and the overall robustness of the hydrogel occluders. The exemplary composition was chosen as an example because it is particularly thermoreversible and in its nonfluid form and at body temperature it stands up well to the forces of blood flow. Diacrylates may also be employed, but the properties of the final product may deviate from what has been observed in use of the exemplary composition.

In one embodiment, in an optional step 130, the modified polymer (such as P407-DVS) can now be combined with the modified protein (in the exemplary scheme, albumin-SH) and stored at about 4 degrees Celsius. In another embodiment, these components can be mixed just prior to delivery to a body vessel of a patient. While both options are viable, in the exemplary scheme being described, the two components will be stored separately and mixed just prior to use.

The reaction between a free thiol and the activated double bond of a vinyl sulfone group creates a covalent bond between the sulfur of the former thiol and a carbon of the vinyl sulfone group. Without wishing to be bound by a particular theory, the covalent binding of various portions of macromolecules such as a polymer and a polypeptide, or crosslinking, creates the network which forms the hydrogel of the inventive disclosure. In this embodiment, crosslinking causes a non-fluid state of a hydrogel to form and to fill more space than the cooled liquid form of the composition or the unreacted precursor solutions. The degree of crosslinking in large part gives the solid or semisolid composition its characteristic hardness (which may not be particularly hard, but harder than each of the precursor compositions or the cooled, liquid form of the composition) and its tackiness and adhesive properties, particularly to the lumens of vessels to be treated. The hydrogel which results may be highly absorbent and a large amount of the internal volume may comprise water or another aqueous solution. This results in a porous, scaffold-type superstructure into which a load to be released (such as, for example, a sclerosant in accordance with the principles of the present invention) can be provided. Hydrogels are generally considered to be colloidal compositions of matter.

In an embodiment where the modified protein or polypeptide and the modified polymer are kept separate until just prior to use, at least one sclerosant is added. In one example, the sclerosant used is STS, and STS is added in an amount that will assure a final concentration of 3% (weight/volume) in the resultant hydrogel. In a step 140 (not shown) of this non-limiting example, STS is added to the albumin-SH solution to form albumin-SH/STS. In another embodiment not illustrated, STS is added to the polymer solution rather than the protein or polypeptide.

In the example, two solutions that are combined to form a thermoreversible therapeutic solution which can comprise an occlusive hydrogel for treatment of varicose and spider veins have the following compositions: the first solution comprises an albumin-SH/STS solution comprising about 0.5 ml deionized water, about 93.75 milligrams (mg) albumin (18.75% weight/volume), and about 45.0 mg STS (9%); and the second solution, which is a P407-DVS solution, comprises about 1.0 ml deionized water and about 187.5 mg P407-DVS (18.75%). When the two solutions are combined prior to use, the resultant solution comprises about 12.5% P407-DVS, about 6.25% Albumin-SH, and about 3% STS. This represents one possible tripartite compound which comprises a thermoreversible hydrogel for occlusion of a blood vessel and delivery of a sclerosant.

In one embodiment, a method of treatment for a patient comprises mixing a sclerosant or sclerosant solution with an activated protein solution, combining this with an activated polymer solution at a temperature below the transition temperature, and delivering the mixture to a vessel to be eliminated via syringe. After treatment is complete, the method of treatment may further comprise using an injection of cold saline or otherwise cooling the affected area (for instance, topically), allowing the hydrogel to return to its liquid state, and extracting the therapeutic occlusive material from the vasculature using a syringe or any other acceptable extraction method.

In another embodiment, any water-soluble drug, compound, or any combination thereof can be combined with the protein/polypeptide solution, with the protein solution, or with both, and can be delivered to a body lumen with or without sclerosant.

The hydrogel of the instant disclosure is substantially free of dimethyl sulfoxide due to its synthetic scheme. It has a transition temperature of about 32 degrees Celsius, the composition being liquid below such temperature and substantially solidified above such temperature. The separate components, or the combined tripartite solution can be maintained at about 4 degrees Celsius so that the mixture remains liquid, and is delivered via a syringe, a catheter, a microcatheter, or any other suitable delivery device, said delivery device also being maintained at about 4 degrees Celsius.

The mixture can be injected into varicose or spider veins. As it approaches body temperature, or about 37 degrees Celsius, it becomes more solid. In one embodiment, the transition temperature is about 32 degrees Celsius.

When the veins to be eliminated are destroyed, there is no longer a need for the patient to retain the occlusive compound in his body. Thus, occlusion affected by the exemplary composition of matter can be temporary and reversible. Temporary might mean that the vessel is occluded on the order of days, or weeks, or months. In one study of the exemplary composition, use of the composition in a rabbit model has illustrated that occlusion of at least a month is possible. The sclerosant is thought to be gradually delivered to the vessel lumen as it diffuses out of the solid hydrogel, constantly irritating and eventually destroying the vessels from the inside. Dead vessels are then absorbed into the surrounding tissue. Cooling via cool saline which is injected. Moreover, it has been shown by studies conducted on a porcine model that the exemplified composition can be removed from a surface vein. To affect removal, injection of cooled saline solution is injected into the body of the patient at a site near the hydrogel occluder. When the hydrogel is cooled to below its transition temperature (about 32 degrees Celsius in the case of the exemplary tripartite composition) the compound liquefies and can then be removed by a syringe or in some cases a vacuum apparatus.

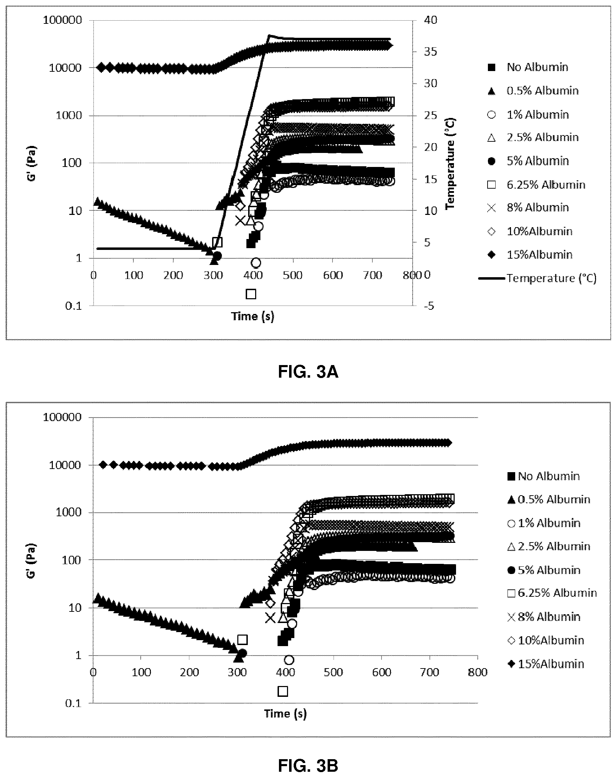

An exemplary study of a series of occlusive compounds in accordance with the principles of the present disclosure is illustrated in FIGs. 3A-3B. Occlusive compounds comprising about 12.5% Poloxamer 407 (actual quantities 12.26%-12.54% inclusive) and about 0% to about 15% weight-to-volume albumin were generated according to the scheme described herein. The percentages weight-to-volume, nominal and actual, of albumin studied were 0% (0%), 0.5% (0.49%), 1% (0.93%), 2.5% (2.46%), 5% (4.96%), 6.25% (6.25%), 8% (7.96%), 10% (10.00%), and 15% (14.96%). Occlusive compounds were mixed and stored at about 4 degrees Celsius, and were then placed in a rheometer having a temperature controller. The storage modulus of each occlusive compound was measured across about 750 seconds. For about 300 seconds, the temperature was maintained at about 4 degrees Celsius, whereupon it was raised by the temperature controller to about 37 degrees Celsius over the course of about 100 seconds (solid line of FIG. 3A.) The temperature was then maintained at about 37 degrees for about 300 seconds. In measuring the storage moduli, values of G' in pascals were obtained.

As can be seen in FIG. 3A and 3B, occlusive compounds lacking albumin had a maximal G' of about 90.06 Pa. Occlusive compound with 0.5% albumin had maximal G' of about 206.7 Pa; 1%, 51.66 Pa; 2.5%, 327.3 Pa; 5%, 323.9 Pa; 6.25%, 1923 Pa; 8%, 559.5 Pa; 10%, 1589 Pa; and 15%, 29050 Pa. However, the occlusive compound with 15% albumin also had a high initial G' of about 10000 Pa. Higher G' may be associated with smaller chance of migration of the occlusive compound, so in the case where about 12.5% of the compound is Poloxamer 407, about 1% to about 15% albumin might be used, more preferably 2.5% to about 10%, most preferably about 6.25% to about 10% albumin.

Other proteins will not behave in the same manner as albumin. A suitable occlusive compound may comprise about 1% to about 50% protein weight-to-volume, or about 2% to about 40% protein, or about 2.5% to about 25% protein, or about 4% to about 20% protein, or about 4.25% to about 15% protein, or about 4.5% to about 10% protein, or about 5% to about 10% protein. The protein may be combined with about 5% to about 25% polymer.

Likewise, different concentrations of polymer, such as Poloxamer 407, may be used. Suitable occlusive compounds may comprise about 5% to about 20% Poloxamer 407, or about 7.5% to about 17.5% Poloxamer 407, or about 10% to about 15% Poloxamer 407, all weight-to-volume. A suitable occlusive compound may comprise about 5% to about 25% polymer weight-to-volume, or about 7.5% to about 22.5% polymer, or about 10% to about 20% polymer, or about 11% to about 15% polymer. The polymer may be combined with about 1% to about 50% protein weight-to-volume to result in the occlusive compound.

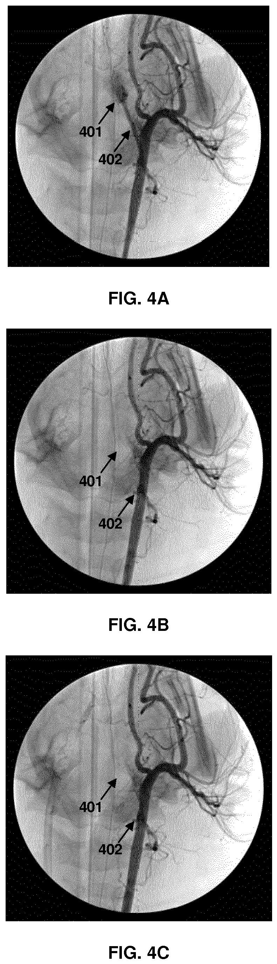

Turning now to FIG. 4A, 4B, and 4C, a series of photographs representing the use of an occlusive composition of the present disclosure is shown. In FIG. 4A, injection of contrast media into the vasculature of a pig is portrayed. The rete mirabile 401, a complex of veins and arteries, is visible, as is the vessel 402 which feeds the rete mirabile. Vessel 402 is the vessel to be occluded. Fluoroscopy of the vasculature in this area, as shown in FIG. 4A, demonstrates that fluid is flowing to all vessels in the area.

Injection of the occlusive compound then occurs. To achieve injection, a microcatheter was advanced toward the rete mirabile via the subclavian artery under fluoroscopy. The two components of the occlusive compound (that is, a first solution including the protein component and a second solution including the non-protein polymer compound), with one of the solutions containing a sclerosant, are mixed via a three-way stopcock immediately prior to injection. The stopcock was connected to the microcatheter, and about 1.0 ml of occlusive compound was injected.

FIG. 4B shows the vasculature about one minute after injection of an albumin-poloxamer 407-divinyl sulfone occlusive compound upstream of the rete mirabile. At this time, contrast media was injected, and the rete mirabile 401 is no longer visible, as blood flow (and therefore flow of contrast media) has been blocked by the occlusive compound. FIG. 4C is similar to FIG. 4B, except in this case injection of contrast media was conducted about five minutes after occlusion. As can be seen in FIG. 4C, no contrast media makes its way to the rete mirabile, which is therefore still occluded,

While the present invention has been described in terms of preferred embodiments, it will be understood, of course, that the invention is not limited thereto since modifications may be made to those skilled in the art, particularly in light of the foregoing teachings.

## Claims

1. An occlusive compound for injection into and occlusion of a body vessel, the occlusive compound comprising:
a polymer comprising 5 percent to 25 percent of the occlusive compound weight-to-volume, a protein comprising a sulfur-bearing amino acid comprising 1 percent to 50 percent of the occlusive compound weight-to-volume, and a sclerosant, the occlusive compound being substantially fluid at a first temperature and substantially non-fluid at a second temperature, the first temperature being lower than the second temperature;
wherein:
the polymer is a nonionic polymer comprising a first chain having a first end and a second end, a second chain having a first end and a second end, and a third chain having a first end and a second end, the second end of the first chain being bound to the first end of the second chain, the second end of the second chain being bound to the first end of the third chain; the first chain and the third chain comprising poly(ethylene oxide) and together comprising about seventy percent of a molecular mass of the polymer; the second chain comprising poly(propylene oxide) and having a molecular mass of about 4000;
the protein comprises an albumin;
the polymer and the protein are mixed and crosslinked; and
the sclerosant comprises sodium tetradecyl sulfate or polidocanol.

2. The occlusive compound of claim 1 wherein the sclerosant comprises sodium tetradecyl sulfate.

3. The occlusive compound of any preceding claim further comprising a water-soluble drug.

4. A composition of matter for injection into and occlusion of a body vessel, the composition comprising:
a nonionic copolymer comprising a first chain having a first end and a second end, a second chain having a first end and a second end, and a third chain having a first end and a second end, the second end of the first chain being bound to the first end of the second chain, the second end of the second chain being bound to the first end of the third chain;
the first chain and the third chain comprising poly(ethylene oxide) and together comprising seventy percent of a molecular mass of the polymer;
the second chain comprising poly(propylene oxide) and having a molecular mass of 4000;
an albumin; and
a sclerosant;
wherein the albumin and the nonionic copolymer are crosslinked for occlusion of the body vessel; and
wherein the sclerosant comprises sodium tetradecyl sulfate or polidocanol.

5. The composition of matter of claim 4 wherein: (i) the sclerosant comprises sodium tetradecyl sulfate; and/or (ii) the nonionic copolymer comprises 12.5% of the composition of matter weight-to-volume and the albumin comprises 6.5% of the composition of matter weight-to-volume.

## Patentansprüche

1. Okklusive Verbindung zum Einspritzen in ein und zur Okklusion eines Körpergefäß(es), wobei die okklusive Verbindung umfasst:
ein Polymer umfassend 5 Gewicht-zu-Volumen-Prozent bis 25 Gewicht-zu-Volumen-Prozent der okklusiven Verbindung, ein Protein, das eine schwefeltragende Aminosäure umfasst, umfassend 1 Gewicht-zu-Volumen-Prozent bis 50 Gewicht-zu-Volumen-Prozent der okklusiven Verbindung, und ein sklerosierendes Mittel, wobei die okklusive Verbindung bei einer ersten Temperatur im Wesentlichen fluid ist und bei einer zweiten Temperatur im Wesentlichen nichtfluid ist, wobei die erste Temperatur niedriger als die zweite Temperatur ist;
wobei:
das Polymer ein nichtionisches Polymer ist, das eine erste Kette mit einem ersten Ende und einem zweiten Ende, eine zweite Kette mit einem ersten Ende und einem zweiten Ende und eine dritte Kette mit einem ersten Ende und einem zweiten Ende umfasst, wobei das zweite Ende der ersten Kette an das erste Ende der zweiten Kette gebunden ist, das zweite Ende der zweiten Kette an das erste Ende der dritten Kette gebunden ist; die erste Kette und die dritte Kette Poly(ethylenoxid) umfassen und zusammen etwa siebzig Prozent der Molekülmasse des Polymers umfassen; die zweite Kette Poly(propylenoxid) umfasst und eine Molekülmasse von etwa 4000 aufweist;
das Protein ein Albumin umfasst;
das Polymer und das Protein gemischt und vernetzt sind; und
das sklerosierende Mittel Natriumtetradecylsulfat oder Polidocanol umfasst.

2. Okklusive Verbindung gemäß Anspruch 1, wobei das sklerosierende Mittel Natriumtetradecylsulfat umfasst.

3. Okklusive Verbindung gemäß einem der vorstehenden Ansprüche, ferner umfassend ein wasserlösliches Arzneimittel.

4. Stoffzusammensetzung zum Einspritzen in ein und zur Okklusion eines Körpergefäß(es), wobei die Zusammensetzung umfasst:
ein nichtionisches Copolymer umfassend eine erste Kette mit einem ersten Ende und einem zweiten Ende, eine zweite Kette mit einem ersten Ende und einem zweiten Ende und eine dritte Kette mit einem ersten Ende und einem zweiten Ende, wobei das zweite Ende der ersten Kette an das erste Ende der zweiten Kette gebunden ist, das zweite Ende der zweiten Kette an das erste Ende der dritten Kette gebunden ist;
die erste Kette und die dritte Kette Poly(ethylenoxid) umfassen und zusammen siebzig Prozent der Molekülmasse des Polymers umfassen;
die zweite Kette Poly(propylenoxid) umfasst und eine Molekülmasse von 4000 aufweist;
ein Albumin; und
ein sklerosierendes Mittel;
wobei das Albumin und das nichtionische Copolymer zur Okklusion des Körpergefäßes vernetzt sind; und
das sklerosierende Mittel Natriumtetradecylsulfat oder Polidocanol umfasst.

5. Stoffzusammensetzung gemäß Anspruch 4, wobei: (i) das sklerosierende Mittel Natriumtetradecylsulfat umfasst; und/oder (ii) das nichtionische Copolymer 12,5 Gewicht-zu-Volumen-% der Stoffzusammensetzung umfasst und das Albumin 6,5 Gewicht-zu-Volumen-% der Stoffzusammensetzung umfasst.

## Revendications

1. Composé occlusif pour une injection dans un vaisseau corporel et l'occlusion d'un vaisseau corporel, le composé occlusif comprenant :
un polymère comprenant 5 pour cent à 25 pour cent du composé occlusif poids-sur-volume, une protéine comprenant un acide aminé portant un soufre comprenant 1 pour cent à 50 pour cent du composé occlusif poids-sur-volume, et un sclérosant, le composé occlusif étant sensiblement fluide à une première température et sensiblement non fluide à une deuxième température, la première température étant inférieure à la deuxième température ;
le polymère étant un polymère non ionique comprenant une première chaîne possédant une première terminaison et une deuxième terminaison, une deuxième chaîne possédant une première terminaison et une deuxième terminaison, et une troisième chaîne possédant une première terminaison et une deuxième terminaison, la deuxième terminaison de la première chaîne étant liée à la première terminaison de la deuxième chaîne, la deuxième terminaison de la deuxième chaîne étant liée à la première terminaison de la troisième chaîne ; la première chaîne et la troisième chaîne comprenant un poly(oxyde d'éthylène) et représentant ensemble environ septante pour cent d'une masse moléculaire du polymère ; la deuxième chaîne comprenant un poly(oxyde de propylène) et possédant une masse moléculaire d'environ 4 000 ;
la protéine comprenant une albumine ;
le polymère et la protéine étant mélangés et réticulés ; et
le sclérosant comprenant du tétradécylsulfate de sodium ou du polidocanol.

2. Composé occlusif selon la revendication 1, le sclérosant comprenant du tétradécylsulfate de sodium.

3. Composé occlusif selon une quelconque revendication précédente comprenant en outre un médicament hydrosoluble.

4. Composition de matière pour une injection dans un vaisseau corporel et l'occlusion d'un vaisseau corporel, la composition comprenant :
un copolymère non ionique comprenant une première chaîne possédant une première terminaison et une deuxième terminaison, une deuxième chaîne possédant une première terminaison et une deuxième terminaison, et une troisième chaîne possédant une première terminaison et une deuxième terminaison, la deuxième terminaison de la première chaîne étant liée à la première terminaison de la deuxième chaîne, la deuxième terminaison de la deuxième chaîne étant liée à la première terminaison de la troisième chaîne ;
la première chaîne et la troisième chaîne comprenant un poly(oxyde d'éthylène) et représentant ensemble septante pour cent d'une masse moléculaire du polymère ;
la deuxième chaîne comprenant un poly(oxyde de propylène) et possédant une masse moléculaire d'environ 4 000 ;
une albumine ; et
un sclérosant ;
l'albumine et le copolymère non ionique étant réticulés pour l'occlusion du vaisseau corporel ; et
le sclérosant comprenant du tétradécylsulfate de sodium ou du polidocanol.

5. Composition de matière selon la revendication 4 : (i) le sclérosant comprenant du tétradécylsulfate de sodium ; et/ou (ii) le copolymère non ionique représentant 12,5 % de la composition de matière poids-sur-volume et l'albumine représentant 6,5 % de la composition de matière poids-sur-volume.
